# EUROPEAN PATENT SPECIFICATION

(11) **EP 2 103 259 B1**
(45) Date of publication and mention of the grant of the patent: **25.01.2012**
(21) Application number: 08153031.3
(22) Date of filing: 19.03.2008
(51) Int. Cl.: A61B 17/02, A61B 19/00, G09B 23/28

(54) **Method and system for determination of a degree of deformity of an anatomical joint**
Verfahren und System zur Bestimmung eines Deformitätsgrades einer anatomischen Verbindungsstelle
Procédé et système pour la détermination d'un degré de difformité d'une articulation anatomique

(43) Date of publication of application: 23.09.2009
(73) Proprietor: BrainLAB AG, 85622 Feldkirchen (DE)
(72) Inventor: Warkentine, Blaine, draper, UT 84020 (US); Spies, Björn, 85622 Feldkirchen (DE)
(74) Representative: Schwabe - Sandmair - Marx

(56) References cited:
- WO-A-01/78015
- US-A1- 2004 030 245
- US-A1- 2007 066 917
- US-A1- 2007 179 626

## Description

### Field of the Invention

The present invention relates to a method and system for assessing or determining a degree of deformity of an anatomical structure joining two limbs and, more particularly, to a method and system for assessing or determining a degree of deformity of an anatomical joint, e. g. a knee joint or elbow joint, as defined in the claims.

### Background of the invention

The assessment or determination of a degree of deformity of a knee joint or any other anatomical structure joining two limbs assist the planning and performing of surgery, alleviates possible complications, and fosters successful rehabilitation.

In cases of mild deformities of a knee joint, the knee joint is suitable for total knee arthroblasty (TKA), for instance.

Determination of structural anatomic features require the acquisition of locations and/or orientations of anatomic objects, such as limbs relative to a spatial coordinate system or operating space.

A locating or tracking device may be used to determine position and/or orientation of an anatomical object in a three-dimensional coordinate system or operating space by means of an array of activated markers attached to the object. By receiving signals emitted by or reflected from the markers with a sensor or sensors in different spatial positions and orientations the position and orientation of the marker array (reference array) and thus the position and/or orientation of the anatomical object to which the marker array is attached to may be determined. The emitted or reflected signals may be electromagnetic waver (e. g. infrared light) or ultra sound waves.

An example of a system for tracking the spatial position and angular orientation of an object may be found in US patent number 5,828,770.

The pose (position and/or orientation) of an object in space is referred to as the pose of the object.

The following prior art documents are cited:
M.E. Nadzadi, MS; J. K. Nielsem, MS; S. B. Murphy, MD, smith and nephew Memphis, TN Intact Knee Passive Kinematics and Ligamentous Stability Measured by a Novel Navigation System;
   and
M.E. Nadzadi, MS; J. K. Nielsem, MS; S. B. Murphy, MD, smith and nephew Memphis, TN A Novel Method for Measuring Intact Knee Joint Laxity and Kinematics Using Computer Assisted Surgical Navigation Tools.

It is an object of the present invention to provide a method and system for preparing surgery of a knee joint or any other anatomical structure joining two limbs by assessing or determining a degree of deformity of the anatomical joint.
This object is achieved by the subject-matter of the independent claims. Preferred embodiments of the present invention are determined in the dependent claims.

US 2007/0066917 A1 shows a method and apparatus for preoperatively or intraoperatively determining prosthetic implant selection and placement to achieve acceptable alignment and spacing of an anatomical structured affected by the prosthetic implant and to achieve acceptable soft tissue balance proximate the prosthetic implant without requiring trial-and-error selection of implant size and placement during surgery. Therein, a general algorithm is suggested for simulating the elastic properties of anatomical structure and comparing to the desired soft tissue balance for certain flexion angles of a knee joint.

US 2007/0179626 A1 shows a method for performing arthroplasty on a joint using a surgical navigation system. Therein, biomechanical properties of the joint are determined and the soft tissue envelope properties are evaluated for the joint. A further focus of the respective invention is to display an interactive view of the joint and to prepare the joint to receive the joint implants as well as install the implants in the prepared joint.

WO 01/78015 A2 shows a computer assisted orthopedic surgery planner software for generation of 3D solid bone models from two or more 2D x-ray images of a patient's bone. That method makes extensive use of free-formed deformation parameters for implementation in Perry's FFD technique.

### Summary of the invention

A method according to the invention for pre-operatively determining a degree of deformity of an anatomical structure joining to limbs may be used to determine a degree of deformity of a knee-joint joining femur and tibia, respectively or of an elbow joint.

A degree of deformity of an anatomical structure joining two limbs comprises the relative angular orientation or angular difference of axis corresponding to the two limbs.

The degree of deformity is measured in angular degrees. An angular difference of a first predetermined angle, e. g. of up to 3° to 4° may be considered as a mild deformity. An angular difference greater than the first predetermined angle but lower than a second predetermined angle, e. g. between 3° and 5° or 4° and 6°may be considered as a moderate deformity.

The deformity may comprise flection deformity and/or various/valgus deformity.

A method according to the invention may comprise the step of providing data representing the pose of an axis of a limb in a reference system in which the limb is fixed.

A reference system in which a limb is fixed, i. e. representing poses of the limb, may be provided by a locating device preferably comprising an array of markers fixedly coupled to the limb. This array is also called reference array.

In order to provide data representing a pose of an axis of a limb in a reference system in which the limb is fixed, an additional locating device (e. g. pointer) or locating devices are brought into contact with the limb in order to object selected poses (e. g. landmarks) determining the axis and measured in the reference system in which the limb is fixed.

The method according to the invention may provide data representing poses of a first limb (e. g. femur) in a first reference system in which the first limb is fixed and of a second axis of the second limb (e. g. tibia) in a reference system in which the second limb is fixed as defined in claim 1 the first and second limb being joined by an anatomical structure.

The method according to the invention further comprises the step of providing force orientation data representing a relative pose between the first and second reference system with the first and second limb in extension, and with an external force being applied to eliminate or reduce the deformity of the anatomical structure. An external force may be applied to the first and second limb in extension in order to at least partially compensate the deformation of the anatomical structure.

A method according to the invention may further comprise the step of determining a relative angular orientation between the first axis of the first limb and the second axis of the second limb based on the force orientation data, said relative angular orientation being referred to as force relative angular orientation. Determining a relative angular orientation between the first and second axis from force orientation data may comprise determining a relation between the first and second reference system with the first and second limb in extension, and with an external force being applied.

In the method according to the invention, a relative angular orientation comprises a sagittal and/or coronal component. Splitting the relative angular orientation into components may simplify the assessment of a degree of deformity of the anatomical structure.

A method according to the invention may further comprise the step of comparing force relative angular orientation with a relative angular orientation which is used as a reference.

The relative angular orientation used as a reference may be a predetermined relative angular orientation which predetermined relative orientation is for instance based on the above mentioned first or second predetermined angle.

A method according to the invention may further obtain the reference relative orientation by the step of providing forceless orientation data representing a relative pose between the first and second reference system with the first and second limb in extension and without an external force being applied. The pose of a limb in a reference system may be represented by the pose of an axis of the limb and/or by poses of landmarks of the limb. An axis of the limb is in particular a characteristical axis of the limb along the extension of the limb.

A method according to the present invention may obtain the reference relative angular orientation by the further step of determining the reference relative angular orientation, i. e. a forceless relative angular orientation, between the poses of the limbs e. g. between the first axis of the first limb and the second axis of the second limb based on forceless orientation data. A method according to the invention may be executed by a computer program loaded by and/or executed on a general purpose computer, a dedicated computer or a computer on a chip.

In a system according to the invention a computer on which a program for executing a method in accordance with the invention is running or loaded may be operatively coupled to a detector for detecting locating devices or reference markers to acquire data representing poses of objects.

### Brief description of the drawings

The foregoing and other features of the invention are hereinafter discussed with reference to the drawings.
Figures 1 and 2 show plan views of an anatomical knee joint subjected to method steps according to the invention.
Figures 3 and 4 show flowcharts illustrating programs, which when running on a computer or loaded on a computer execute methods according to the invention.
Figure 5 diagrammatically shows a computer system on which a program or programs according to the invention may be running or loaded.

### Detailed description

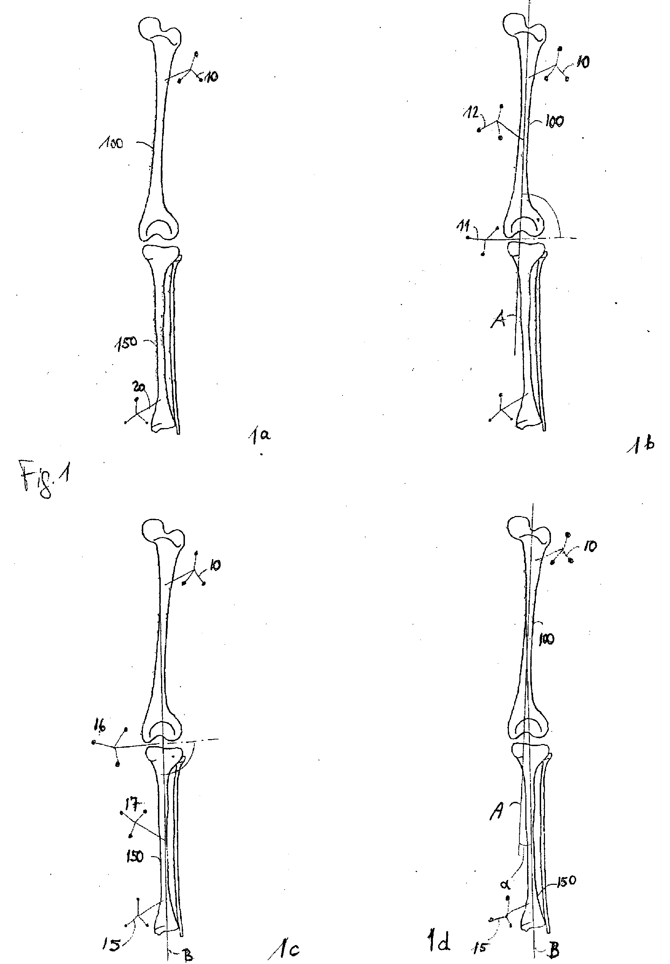

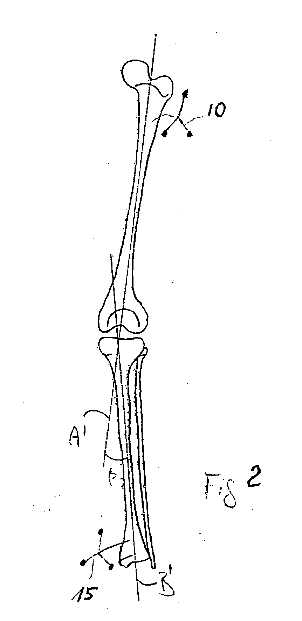

Figures 1 and 2 show plan views of a knee joint joining femur 100 and tibia 150.

The assessment or determination of a degree of deformity of an anatomical structure joining two limbs, particularly of a knee joint, starts with the establishment with a first and second reference system (coordinate system) in a surrounding space such as the operating space.

In the first reference system, the femoral system, the femur is fixed. This means that the reference system follows dislocations or movements of the femur in the surrounding space or operating space.

This following or tracking may be achieved by a locating or tracking device 10 also called a marker array or reference array, which is fixedly attached to the femur 100, c. g. clamped or otherwise fastened to the femur as shown in Figure 1a. As the femur 100 or any other limb 100 of a patient to which a marker array 150 is attached moves or is moved the marker array 150 describes a corresponding movement. A marker array 150 preferably comprises an array of elements or markers which can be activated and/or located by a remote sensor. The sensor preferably detects a current pose in a surrounding space or operating space.

Once a tracking device 10 is fixed to the femur selected positions and/or orientations may be acquired at one or more tracking devices which are temporarily brought into the respective positions and/or orientations relative to the femur.

Reference is now made to Figure 1 b. In order to obtain data representing a relative pose of the longitudinal femoral axis in the femoral reference system, a pointer 11 (comprising markers) is temporarily placed onto a landmark, e. g. the center of the distal femoral cortex in an orientation perpendicular to the femoral axis and the pose of the pointer in a surrounding space or operating space is acquired by a remote sensor. Since the relative location between the tip of the pointer which touches the landmark and the markers of the pointer is known, the location of the landmarks may be inferred from the pose of the pointer.

Simultaneously a pose of the marker array 10 which defines the femoral reference system is acquired.

In addition to the pose of the distal femoral cortex center a pose of a pointer 12 is temporarily placed at another landmark, e. g. midway on the longitudinal femoral axis and a corresponding pose is acquired.

The pose of a pointer 11 at the center of the femoral cortex and the pose of a pointer 12 midway on the femoral axis need not to be acquired at the same time as long as the corresponding pose of the femoral reference system is acquired together with any of these poses. In this way the relation pose between the marker array 11 and the landmarks is acquired. This allows to define the pose of the femoral axis in the femoral reference system.

An alternative way of obtaining data representing a pose of the longitudinal femoral axis in the femoral reference system would be to acquire a position of the center of the cortex of the femur 100 and/or two neighbouring points on an axis, preferably a saggital or coronal axis, through the distal cortex of the femur (not shown). With the poses of selected points (e. g. landmarks) on the femoral axis in the femoral reference system the pose of the femoral axis itself in the femoral system can be determined.

Reference is now made to Figure 1c. In order to obtain data representing a relative pose of the longitudinal tibial axis in the tibial reference system, a pointer 16 is temporarily placed onto the center of the proximal tibial cortex in an orientation perpendicular to the tibial axis and the pose of the pointer in a surrounding space or operating space is acquired by a remote sensor.

Simultaneously a pose of the marker array 15 which defines the tibial reference system is acquired.

In addition to the pose of the proximal tibial cortex center a pose of a pointer 17 temporarily placed midway on the longitudinal tibial axis and a corresponding pose of the maker array 15 defining the tibial reference system are acquired.

The pose of a pointer 16 at the center of the tibial cortex and the pose of a pointer midway on the tibial axis need not to be acquired at the same time as long as the corresponding pose of the tibial reference system is acquired together with any of these poses.

An alternative way of obtaining data representing a pose of the longitudinal tibial axis in the tibial reference system would be to acquire a position of the center of the cortex of the tibia 150 and/or to neighbouring points on an axis, preferably on a saggital or coronal axis, through the proximal cortex of the tibia (not shown). With these poses of selected points on the tibial axis in the tibial reference system the pose of the tibial axis itself in the tibial system can be determined.

With the markers array 10 and 15 remaining fixedly attached to femur 100 and tibia 150 data representing a pose of the femoral axis and a pose of the tibial axis, respectively, can be determined either in the surrounding space or relative to each other. The latter data represent a relative pose between the femoral and tibial preference system.

The degree of a pre-operative deformity of a knee joint may be considered as the deformity of femur and tibia in extension that can be eliminated or at least reduced by applying an external force. The external force may preferably compensate for a varus and/or valgus deformity.

With femur and tibia in extension and with an external force applied to eliminate or reduce a deformity of the knee joint orientation data representing a relative pose between the femoral axis A and the tibial axis B can be obtained. The orientation data are referred to as force orientation data.

Finally, as shown in Figure 1d, force orientation data determine a relative angular orientation α between the femoral axis A and the tibial axis B.

The relative angular of orientation α may comprise a respective saggital and/or coronal component.

By comparing the force relative angular orientation α with a reference relative angular orientation a degree of deformity of an anatomical structure joining two limbs, such as a knee joint, may be determined.

The reference relative angular orientation may be a predetermined relative angular orientation. By comparing the force relative angular orientation α as shown in Figure 1d with a particular reference relative angular orientation a still more accurate assessment of a degree of deformity of an anatomical structure joining two limbs, such as a knee joint may be performed.

This particular reference relative angular orientation may be determined as follows:

With the markers arrays 10 and 15 preferably still in place, i. e. the marker arrays determining a femoral and a tibial reference system, respectively, other relative poses between the femoral and tibial reference system and consequently other relative angular orientations between the femoral and tibial axis may be acquired.

Alternatively, the steps described above with reference to Figures 1a to 1c could be repeated on another point in time for instance, in order to provide a tibial and a femoral reference system and poses of femoral and tibial axes in the respective reference sytem.

As shown in Figure 2 with femur 100 and tibia 150 in extension, and without an external force being applied orientation data representing a relative pose between the femoral axis A' and tibial axis B' can be obtained. These orientation data are referred to as forceless orientation data. As shown in Figure 2 forceless orientation data determine a relative angular orientation β between the femoral axis A' and the tibial axis B', referred to as forceless relative angular orientation.

A degree of deformity of an anatomical structure joining two limbs, such as a knee joint may be preferably determined by comparing the force relative angular orientation α with the forceless relative angular orientation β.

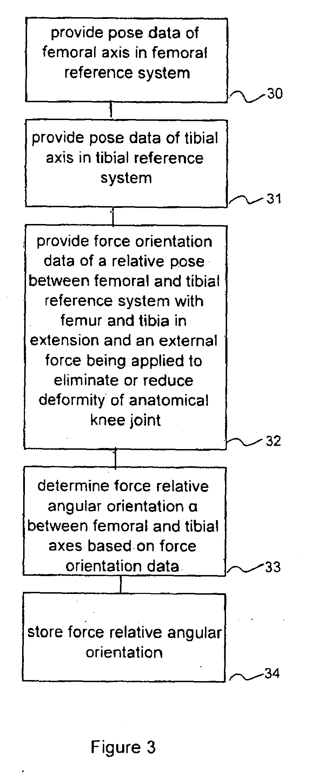

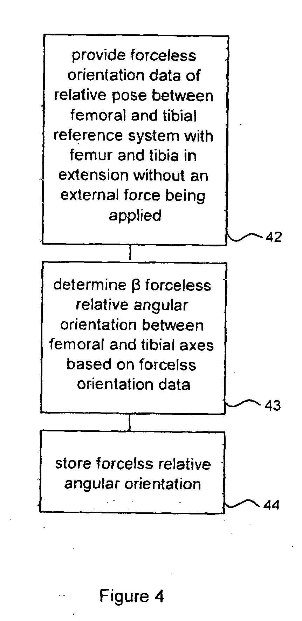

Figures 3 and 4 show flowcharts illustrating programs, which when running on a computer or loaded on a computer execute the methods described above.

Referring to Figure 3, pose data of a femoral axis in a femoral reference system is provided in step 30. Pose data of a tibial axis in a tibial reference system is provided in step 31. Force orientation data of a relative pose between the femoral and the tibial reference system with femur and tibia in extension and an external force being applied to eliminate or reduce a deformity of an anatomical knee joint is provided in step 32.

A force relative angular orientation α between a femoral and tibial axis based on force orientation data is determined in step 33.

Optionally, force relative angular orientation data may be stored in an appropriate data storage device in step 33.

Reference is now made to Figure 4. Forceless orientation data of a relative pose between a femoral and a tibial reference system with femur and tibia in extension without an external force being applied are provided in step 42.

A forceless relative angular orientation β between a femoral and a tibial axis based on forceless orientation data is determined in step 43.

Optionally, forceless relative angular orientation data are stored in a suitable storage device in step 44.

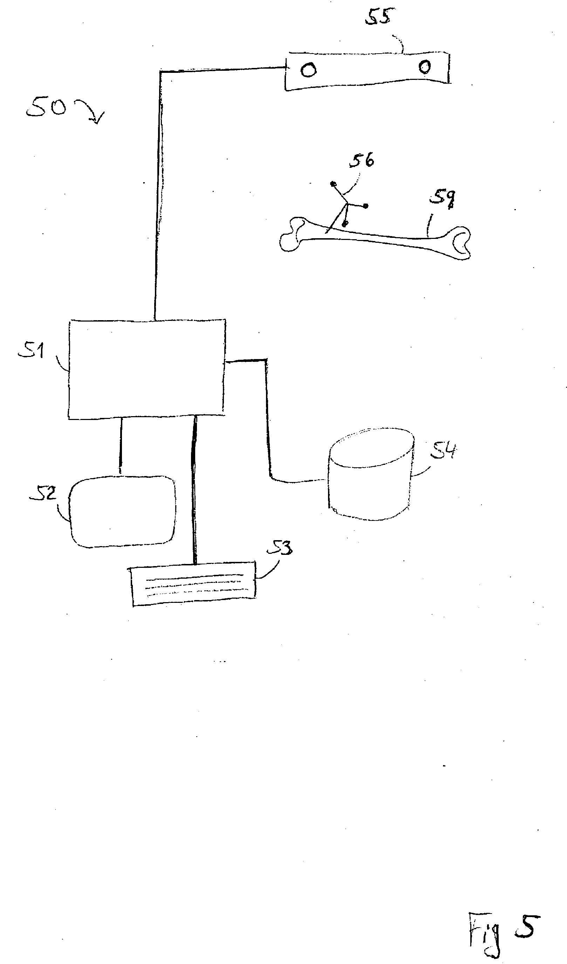

Figure 5 shows a system 50 comprising a computer on which the program or programs described above may be running or loaded. Operatively coupled to the computer 51 is a detector 55 for detecting locating devices representing poses of objects, preferably anatomical element S59. The computer 51 may further comprise a display 52, a tactile input device such as a keyboard 53 and/or a mouse (not shown) and a storage device 54.

## Claims

1. Method of operating a computer for pre-operatively determining a degree of deformity of an anatomical structure joining a first (100) and second (150) limb, the method comprising the steps:
a) providing data representing the pose of a first axis (A) of the first limb (100) in a first reference system in which the first limb is fixed;
b) providing data representing the pose of a second axis (B) of the second limb (150) in a second reference system in which the second limb is fixed;
c) providing force orientation data representing a relative pose between the first and second reference system with the first (100) and second (150) limb in extension, and with an external force being applied to eliminate or reduce the deformity of the anatomical structure; and
d) determining a relative angular orientation (α) between the first axis of the first limb and the second axis of the second limb based on the force orientation data, said relative angular orientation being referred to as force relative angular orientation,
whereby
the relative angular orientation comprises a respective sagittal and coronal component.

2. Method according to claim 1, wherein the anatomical structure joining a first and second limb comprises a knee joint, the first and second limb comprises femur and tibia, respectively, and the angular deformity comprises a flexion deformity and/or a varus/valgus deformity.

3. Method according to any of the preceding claims further comprising:
recording data representing force relative angular orientation.

4. Method according to any of claims 1 to 3 further comprising:
comparing force relative angular orientation with a reference relative angular orientation.

5. Method according to claim 3 wherein the reference relative angular orientation comprises a predetermined relative angular orientation.

6. Method according to claim 4, wherein the reference relative angular orientation is obtained by
e) providing forceless orientation data representing a relative pose between the first and second reference system with the first and second limb in extension and without an external force being applied; and
f) determining a forceless relative angular orientation as reference relative angular orientation between the first axis of the first limb and the second axis of the second limb based on forceless orientation data.

7. Method according to claim 6 further comprising:
recording data representing forceless relative angular orientation.

8. Method according to claim 6 or 7, wherein the step of comparing comprises obtaining a difference vector between the force relative angular orientation and the forceless relative angular orientation in order to obtain a correction vector suitable to virtually correct a pre-operative deformity of the anatomical structure joining the first and second limb.

9. Program, which when running on a computer or loaded on a computer executes the method according to any of the preceding claims.

10. System comprising:
a computer on which the program of the preceding claim is running or loaded, and
a detector, operatively coupled to the computer, for detecting locating devices or reference markers representing poses.

## Patentansprüche

1. Verfahren zum Bedienen eines Computers, um einen Deformierungsgrad einer anatomischen Struktur, die ein erstes (100) und ein zweites (150) Glied verbindet, präoperativ zu bestimmen, wobei das Verfahren folgende Schritte umfasst:
a) Bereitstellen von Daten, die die Stellung einer ersten Achse (A) des ersten Glieds (100) in einem ersten Bezugssystem repräsentieren, in dem das erste Glied ortsfest ist;
b) Bereitstellen von Daten, die die Stellung einer zweiten Achse (B) des zweiten Glieds (150) in einem zweiten Bezugssystem repräsentieren, in dem das zweite Glied ortsfest ist;
c) Bereitstellen von Orientierungsdaten bei Kraftaufbringung, die eine relative Stellung zwischen dem ersten und zweiten Bezugssystem bei gestrecktem ersten (100) und zweiten (150) Glied mit Aufbringung einer äußeren Kraft zur Eliminierung oder Reduzierung der Deformierung der anatomischen Struktur repräsentieren, und
d) Bestimmen einer relativen Winkelorientierung (α) zwischen der ersten Achse des ersten Glieds und der zweiten Achse des zweiten Glieds basierend auf den Orientierungsdaten bei Kraftaufbringung, wobei diese relative Winkelorientierung als relative Winkelorientierung bei Kraftaufbringung bezeichnet wird,
wobei die relative Winkelorientierung jeweils eine sagittale und eine koronale Komponente umfasst.

2. Verfahren nach Anspruch 1, bei dem die anatomische Struktur, die ein erstes und ein zweites Glied verbindet, ein Kniegelenk umfasst, wobei das erste bzw. zweite Glied ein Femur bzw. eine Tibia umfasst, und wobei die Winkeldeformierung eine Flexions-Deformierung und/oder eine Varus/Valgus-Deformierung umfasst.

3. Verfahren nach einem der vorhergehenden Ansprüche, das ferner folgenden Schritt umfasst:
Aufzeichnen von Daten, die die relative Winkelorientierung bei Kraftaufbringung repräsentieren.

4. Verfahren nach einem der Ansprüche 1 bis 3, das ferner folgenden Schritt umfasst:
Vergleichen der relativen Winkelorientierung bei Kraftaufbringung mit einer relativen Referenz-Winkelorientierung.

5. Verfahren nach Anspruch 3, bei dem die relative Referenz-Winkelorientierung eine vorbestimmte relative Winkelorientierung umfasst.

6. Verfahren nach Anspruch 4, bei dem die relative Referenz-Winkelorientierung durch folgende Schritte gewonnen wird:
e) Bereitstellen von Orientierungsdaten ohne Kraftaufbringung, die eine relative Stellung zwischen dem ersten und zweiten Bezugssystem bei gestrecktem ersten und zweiten Glied ohne Aufbringung einer äußeren Kraft repräsentieren; und
f) Bestimmen einer relativen Winkelorientierung ohne Kraftaufbringung als relative Referenz-Winkelorientierung zwischen der ersten Achse des ersten Glieds und der zweiten Achse des zweiten Glieds basierend auf den Orientierungsdaten ohne Kraftaufbringung.

7. Verfahren nach Anspruch 6, das ferner folgenden Schritt umfasst:
Aufzeichnen von Daten, die die relative Winkelorientierung ohne Kraftaufbringung repräsentieren.

8. Verfahren nach Anspruch 6 oder 7, bei dem der Vergleichsschritt darin besteht, einen Differenzvektor zwischen der relativen Winkelorientierung bei Kraftaufbringung und der relativen Winkelorientierung ohne Kraftaufbringung zu gewinnen, um einen Korrekturvektor zu erhalten, der dafür geeignet ist, eine präoperative Deformierung der anatomischen Struktur, die das erste und das zweite Glied verbindet, virtuell zu korrigieren.

9. Programm, das, wenn es auf einem Computer läuft oder in einen Computer geladen wird bzw. ist, das Verfahren nach einem der vorhergehenden Ansprüche ausführt.

10. System, mit:
einem Computer, auf dem das Programm nach dem vorhergehenden Anspruch läuft oder in den das Programm nach dem vorhergehenden Anspruch geladen wird bzw. ist, und
einem Detektor, der bedienungstechnisch mit dem Computer gekoppelt ist, um Ortungsgeräte oder Referenzmarker, die Stellungen repräsentieren, zu detektieren.

## Revendications

1. Procédé d'exploitation d'un ordinateur pour déterminer d'une manière pré-opératoire un degré de déformation d'une structure anatomique articulant un premier (100) et un second (150) segments d'un membre, le procédé comportant les étapes consistant à :
a) obtenir des données représentant l'attitude d'un premier axe (A) du premier segment (100) dans un premier système de référence dans lequel le premier segment est fixe ;
b) obtenir des données représentant l'attitude d'un second axe (B) du second segment (150) dans un second système de référence dans lequel le second segment est fixe ;
c) obtenir des données d'orientation en présence d'une force représentant une attitude relative entre les premier et second systèmes de référence avec les premier (100) et second (150) membres en extension, et une force extérieure étant appliquée pour éliminer ou réduire la déformation de la structure anatomique ; et
d) déterminer, d'après les données d'orientation en présence d'une force, une orientation angulaire relative (α) entre le premier axe du premier segment et le second axe du second segment, ladite orientation angulaire relative étant appelée orientation angulaire relative en présence d'une force,
l'orientation angulaire relative ayant des composantes sagittale et coronale respectives.

2. Procédé selon la revendication 1, dans lequel la structure anatomique articulant un premier et un second segments d'un membre consiste en une articulation du genou, les premier et second segments étant respectivement constitués par un fémur et un tibia et la déformation angulaire consistant en une déformation en varus/valgus.

3. Procédé selon l'une quelconque des revendications précédentes, comportant :
l'enregistrement de données représentant l'orientation angulaire relative en présence d'une force.

4. Procédé selon l'une quelconque des revendications 1 à 3, comportant en outre :
une comparaison de l'orientation angulaire relative en présence d'une force avec une orientation angulaire relative de référence.

5. Procédé selon la revendication 3, dans lequel l'orientation angulaire relative de référence consiste en une orientation angulaire relative prédéterminée.

6. Procédé selon la revendication 4, dans lequel l'orientation angulaire relative de référence s'obtient en
e) obtenant des données d'orientation en l'absence de force, représentant une pose relative entre les premier et second systèmes de référence avec les premier et second segments en extension et sans application d'une force extérieure ; et
f) déterminant, d'après les données d'orientation en l'absence de force, une orientation angulaire relative en l'absence de force comme orientation angulaire relative de référence entre le premier axe du premier segment et le second axe du second membre.

7. Procédé selon la revendication 6, comportant en outre :
l'enregistrement de données représentant l'orientation angulaire relative en l'absence de force.

8. Procédé selon la revendication 6 ou 7, dans lequel l'étape de comparaison consiste à obtenir un vecteur de différence entre l'orientation angulaire relative en présence d'une force et l'orientation angulaire relative en l'absence d'une force afin d'obtenir un vecteur de correction convenant pour corriger virtuellement un déformation pré-opératoire de la structure anatomique articulant les premier et second segments.

9. Programme qui, lorsqu'il est lancé dans un ordinateur ou chargé dans un ordinateur, exécute le procédé selon l'une quelconque des revendications précédentes.

10. Système comportant :
un ordinateur dans lequel le programme selon la revendication précédente est lancé ou chargé, et
un détecteur, coopérant avec l'ordinateur, pour détecter des dispositifs de localisation ou des repères de référence représentant des attitudes.
